(19) Europäisches Patentamt
European Patent Office
Office européen des brevets

(11) Numéro de publication : **0 434 516 A2**

(12) # DEMANDE DE BREVET EUROPEEN

(21) Numéro de dépôt : **90403587.0**

(22) Date de dépôt : **14.12.90**

(51) Int. Cl.5 : **C07C 47/575**, C07C 43/20, C07C 65/21, C07C 69/92, C07C 217/82, C07C 233/54, C07D 213/63, C07D 333/32, C07C 41/16, C07C 45/64

(30) Priorité : **20.12.89 FR 8917212**
**09.11.90 FR 9013903**

(43) Date de publication de la demande : **26.06.91 Bulletin 91/26**

(84) Etats contractants désignés : **AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Huet, Michel**
**63, boulevard Eugène Réguillon**
**F-69100 Villeurbanne (FR)**
Inventeur : **Nobel, Dominique**
**7, rue de Saint-Cloud**
**F-69007 Lyon (FR)**
Inventeur : **Viehe, Heinz Gunther**
**Clos du Paradis No. 7, Grand Manil**
**B-1300 Limal (BE)**

(74) Mandataire : **Dutruc-Rosset, Marie-Claude et al**
**RHONE-POULENC CHIMIE Service Brevets Chimie 25, Quai Paul Doumer**
**F-92408 Courbevoie Cédex (FR)**

(54) **Procédé de préparation de composés aromatiques mono- ou polyalkoxylés.**

(57) La présente invention a pour objet un procédé de préparation de composés aromatiques mono- ou polyalkoxylés à partir de composés aromatiques porteurs d'au moins un atome d'halogène.

L'invention est relative à un procédé de préparation de composés aromatiques alkoxylés qui consiste à faire réagir un composé aromatique porteur d'au moins un atome d'halogène avec un alcoolate de métal alcalin ou alcalino-terreux, en présence d'un catalyseur au cuivre et qui est caractérisé par le fait que l'on opère en présence d'un co-catalyseur qui est un composé insaturé choisi parmi les isocyanures organiques, les isocyanates et les esters des alcyne-dioïques.

Dans une variante d'exécution, l'invention vise également la réaction du produit obtenu après alkoxylation et porteur d'un groupe hydroxyle, avec un agent d'alkylation.

L'invention est parfaitement bien adaptée pour la préparation du triméthoxy-3,4,5 benzaldéhyde et du diméthoxy-3,4 benzaldéhyde.

EP 0 434 516 A2

# PROCEDE DE PREPARATION DE COMPOSES AROMATIQUES MONO- OU POLYALKOXYLES

La présente invention a pour objet un procédé de préparation de composés aromatiques mono- ou polyalkoxylés à partir de composés aromatiques porteurs d'au moins un atome d'halogène.

L'invention concerne également un procédé de préparation de composés aromatiques polyalkoxylés à partir de composés aromatiques, porteurs d'au moins un atome d'halogène et d'au moins un groupe hydroxyle.

L'invention vise plus particulièrement la préparation de triméthoxy-3,4,5 benzaldéhyde à partir de bromo-5 hydroxy-4 méthoxy-3 benzaldéhyde dénommé couramment "bromo-5 vanilline" et de diméthoxy-3,4 benzaldéhyde appelé "vératraldéhyde" à partir de bromo-3 hydroxy-4 benzaldéhyde.

Dans l'exposé qui suit de la présente invention, on entend par "composé aromatique", la notion classique d'aromaticité telle que définie dans la littérature, notamment par Jerry MARCH - Advanced Organic Chemistry, 3ème édition, John Wiley and Sons, 1985 p. 37 et suivantes.

On définit par "composé aromatique polyalkoxylé", un composé aromatique porteur d'au moins deux radicaux alkoxy.

Il est connu selon le brevet GB-A-2 089 672 d'introduire un ou plusieurs substituants alkoxy ou aralkoxy sur un noyau aromatique, en faisant réagir un composé mono- ou polyhalogéné avec un alcoolate de métal alcalin ou alcalino-terreux, en présence d'un catalyseur constitué d'un ester de l'acide formique dénommé "formiate" et d'un sel cuivreux. Dans ce procédé, le formiate utilisé est essentiellement le formiate de méthyle. Une difficulté majeure pour la mise en oeuvre d'un tel procédé réside dans l'utilisation du formiate de méthyle. En effet, ledit réactif n'est pas d'une manipulation aisée car c'est un produit très volatil à température ambiante et pose donc des problèmes de sécurité. Par ailleurs, il est à noter que dans le cas de la méthoxylation, par exemple, du bromobenzène par le méthylate de sodium, en présence de formiate de méthyle et de bromure cuivreux, la réaction est longue et incomplète car 44% de bromobenzène n'ont pas réagi.

De plus, lors de la méthoxylation de composés aromatiques halogénés porteurs également d'une fonction aldéhyde, en particulier le bromo-5 hydroxy-4 méthoxy-3 benzaldéhyde, par réaction avec du méthylate de sodium en présence des catalyseur et co-catalyseur précités, la réaction est également très lente et il est nécessaire dans ce procédé de protéger la fonction aldéhyde en la transformant en acétal.

Un des objectifs de la présente invention est de fournir un procédé d'alkoxylation de composés aromatiques mono- ou polyhalogénés qui permette d'éviter les inconvénients précités.

Un autre objectif de la présente invention est que ledit procédé convienne également pour les composés aromatiques halogénés porteurs d'une fonction aldéhyde sans nécessité de protéger la fonction aldéhyde.

Enfin, un autre objectif de l'invention est que le procédé de l'invention soit tel qu'il permette d'effectuer à la suite, la réaction d'alkylation des composés aromatiques porteurs à la fois d'un groupe alkoxy et d'un groupe hydroxyle obtenus après alkoxylation, sans qu'il soit nécessaire de changer de réacteur.

Il a maintenant été trouvé qu'il était possible d'effectuer dans de bonnes conditions, la réaction d'alkoxylation de composés aromatiques mono- ou polyhalogénés, éventuellement porteurs d'une fonction aldéhyde, en présence d'un catalyseur au cuivre, si l'on opérait en présence d'une quantité efficace d'un co-catalyseur choisi parmi les isocyanures organiques, les isocyanates et les esters des alcyne-dioïques.

Il a également été mis en évidence qu'il était possible en fin de réaction d'alkoxylation, d'enchaîner dans le même milieu réactionnel, une réaction d'alkylation, sans opération supplémentaire.

L'objet de la présente invention est donc un procédé général de préparation de composés aromatiques polyalkoxylés de formule générale (I) :

$$(R_1-O)_m - Ro - (O-R)_n \qquad (I)$$

dans laquelle :

- m et n sont des nombres entiers,
  - n est un nombre entier supérieur ou égal à 1,
  - m est un nombre entier supérieur ou égal à 0,
  - la somme m + n étant comprise, de préférence, entre 1 et 6,
- Ro représente un radical cyclique aromatique ayant au moins 5 atomes dans le cycle, éventuellement substitué, et représentant au moins l'un des radicaux suivants :
  - un radical carbocyclique aromatique, monocyclique ou polycyclique,
  - un radical hétérocyclique aromatique, monocyclique ou polycyclique comportant au moins un des hétéroatomes O, N et S,
- R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

– $R_1$ est un radical alkyle, linéaire ou ramifié ayant de 1 à 6 atomes de carbone,

procédé qui consiste à faire réagir :

- un composé aromatique porteur d'au moins un atome d'halogène et, éventuellement, d'un groupe hydroxyle répondant à la formule générale (II) :

$$(HO)_m - Ro - (X)_n \qquad (II)$$

dans laquelle :

– m et n sont des nombres entiers,
  - n est un nombre entier supérieur ou égal à 1,
  - m est un nombre entier supérieur ou égal à 0,
  - la somme m + n étant comprise, de préférence, entre 1 et 6,

– X représente un atome d'iode, de brome ou de chlore,

– $R_o$ représente un radical cyclique aromatique ayant au moins 5 atomes dans le cycle, éventuellement substitué, et représentant au moins l'un des radicaux suivants :
  - un radical carbocyclique aromatique, monocyclique ou polycyclique,
  - un radical hétérocyclique aromatique, monocyclique ou polycyclique comportant au moins un des hétéroatomes O, N et S, - et un alcoolate de métal alcalin ou alcalino-terreux de formule générale (III) :

$$M^{w+} [ O - R ]_w^- \qquad (III)$$

dans laquelle :

– M représente un métal alcalin ou alcalino-terreux,

– w représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique- ou arylaliphatique, saturé ou insaturé, linéaire ou ramifié,

en présence d'un catalyseur au cuivre qui est le cuivre et/ou un composé du cuivre,

procédé caractérisé par le fait que l'on opère en présence d'une quantité efficace d'un co-catalyseur qui est un composé insaturé choisi parmi les isocyanures organiques, les isocyanates et les esters des alcyne-dioïques.

Dans le présent texte, on désigne d'une manière simplifiée par radicaux alkoxy, des radicaux du type R-O— dans lesquels R représente aussi bien un radical aliphatique saturé ou insaturé qu'un radical cycloaliphatique saturé ou insaturé et qu'un radical aliphatique porteur d'un carbocycle saturé, insaturé ou aromatique.

Dans tous les cas, la substitution de l'atome d'halogène par le radical R-O— est dénommé ci-après "réaction d'alkoxylation", quelle que soit la nature du radical R.

Le terme "alcoolate" est utilisé dans le présent texte de manière générique et désigne également les aralkoxydes métalliques.

L'invention s'applique plus particulièrement aux composés aromatiques porteurs d'au moins un atome d'halogène et éventuellement d'un groupe hydroxyle de formule (II) dans laquelle le radical $R_o$ symbolise :

1° - un radical carbocyclique aromatique, monocyclique ou polycyclique.

Par "radical carbocyclique polycyclique", on entend :
  - un radical constitué par au moins 2 carbocycles aromatiques et formant entre eux des systèmes ortho ou ortho et péricondensés,
  - un radical constitué par au moins 2 carbocycles dont l'un seul d'entre eux est aromatique et formant entre eux des systèmes ortho ou ortho et péricondensés.

2° - un radical hétérocyclique aromatique, monocyclique ou polycyclique.

Par "radical hétérocyclique polycyclique", on définit :
  - un radical constitué par au moins 2 hétérocycliques contenant au moins un hétéroatome dans chaque cycle dont au moins l'un des deux cycles est aromatique et formant entre eux des systèmes ortho ou ortho et péricondensés,
  - un radical constitué par au moins un cycle hydrocarboné et au moins un hétérocycle dont au moins l'un des cycles est aromatique et formant entre eux des systèmes ortho ou ortho et péricondensés.

3° - un radical divalent constitué par un enchaînement de groupements, tels que définis aux paragraphes 1 et/ou 2 liés entre eux :

- par un lien valentiel,
- par un radical alkylène ou alkylidène ayant de 1 à 4 atomes de carbone, de préférence un radical méthylène ou isopropylidène,
- par l'un des groupes suivants :

$$-O- \ , \quad -CO- \ ,$$

$$-S- \ , \quad -SO- \ , \quad -SO_2- \ ,$$

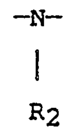 , 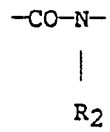

dans ces formules, $R_2$ représentant un atome d'hydrogène ou un radical alkyle ayant de 1 à 4 atomes de carbone, un radical cyclohexyle ou phényle.

Comme exemples de radicaux listés sous 1° à 3°, on peut citer :

1° - les radicaux phényle, tolyle, xylyle ; les radicaux naphtyle, anthryle,

2° - les radicaux furyle, pyrrolyle, thiényle, isoxazolyle, furazannyle, isothiazolyle, imidazolyle, pyrazolyle, pyridyle, pyridazinyle, pyrimidinyle ; les radicaux quinolyle, naphtyridinyle, benzofurannyle, indolyle.

3° - les radicaux biphényle, méthylène-1,1′ biphényle, isopropylidène-1,1′-biphényle, oxy-1,1′ biphényle, imino-1,1′ biphényle.

Les composés préférés sont ceux répondant à la formule (II) dans laquelle le radical $R_o$ représente un noyau benzénique.

Comme mentionné précédemment, le radical $R_o$ qui est un radical aromatique porteur d'au moins un atome d'halogène, peut être également porteur d'un ou plusieurs autres substituants qui peuvent être un autre groupe hydroxyle ou un autre atome d'halogène ou de toute autre nature dans la mesure où ils ne gênent pas la réaction. Généralement, par plusieurs substituants, on définit moins de quatre substituants sur un noyau aromatique.

Comme exemples de substituants donnés à titre illustratif et sans caractère limitatif, on peut mentionner l'un des groupes ou fonctions suivantes :

- un radical de formule $-R_3$-OH dans laquelle le radical $R_3$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,
- un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
- un radical alcényle linéaire ou ramifié ayant de 2 à 6 atomes de carbone, de préférence, de 2 à 4 atomes de carbone, tel que vinyle, allyle,
- un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
- un groupe —CHO,
- un groupe acyle ayant de 2 à 6 atomes de carbone,
- un radical de formule $-R_3$-COOH, $R_3$ ayant la signification donnée précédemment,
- un radical de formule $-R_3$-$COOR_4$ dans laquelle $R_3$ a la signification donnée précédemment et $R_4$ représente un radical alkyle, linéaire ou ramifié, ayant de 1 à 6 atomes de carbone,
- un radical de formule $-R_3$-$NH_2$ avec un groupe $NH_2$ N-protégé, $R_3$ ayant la signification donnée précédemment,
- un radical de formule $-R_3$-$N(R_5)_2$ dans laquelle $R_3$ a la signification donnée précédemment et les radicaux $R_5$ identiques ou différents représentent un atome d'hydrogène ou un radical alkyle, linéaire ou ramifié, ayant de 1 à 6 atomes de carbone,
- un radical de formule $-R_3$-CO-$N(R_5)_2$, $R_3$ et $R_5$ ayant la signification donnée précédemment,
- un radical de formule $-R_3$-Z dans laquelle $R_3$ a la signification donnée précédemment et Z symbolise un atome d'halogène X, tel que précédemment défini, un atome de fluor ou un groupe $CF_3$.

S'il y a présence de substituants sur le cycle aromatique, il y a lieu de veiller que celui-ci n'interfère pas au niveau du produit désiré.

De même, lors de la présence d'une fonction amine primaire, il peut être nécessaire de la N-protéger par un groupe protecteur, par exemple acyle, et plus spécialement acétyle.

Si le cycle est porteur d'un groupe du type —$R_3$-$COOR_4$, le groupe $R_4$ sera échangé par le groupe R provenant de l'alcoolate de métal alcalin ou alcalino-terreux, si $R_4$ et R sont des groupes alkyles de nature différente.

Si le cycle est porteur d'une fonction acide du type —$R_3$-COOH, le groupe sera salifié et entraînera donc une consommation supplémentaire de l'alcoolate utilisé.

Lorsque le cycle aromatique est porteur d'une chaîne aliphatique latérale avec présence d'un atome d'halogène X, celui-ci peut être substitué également par le groupe R-O— provenant de l'alcoolate de métal alcalin ou alcalino-terreux et il y aura donc lieu d'en tenir compte pour la détermination des quantités de réactifs à mettre en oeuvre.

Comme exemples de substituants cités à titre préférentiel et sans caractère limitatif, on peut mentionner:
- un ou plusieurs radicaux alkyle ayant de 1 à 4 atomes de carbone,
- un ou plusieurs radicaux alkoxy ayant de 1 à 4 atomes de carbone,
- un ou plusieurs radicaux hydroxyle.

A titre d'exemples de composés aromatiques porteurs d'au moins un atome d'halogène répondant à la formule générale (II), on peut citer plus particulièrement :
- le bromobenzène
- les dibromobenzènes
- les tribromobenzènes
- le tétrabromo-1,2,4,5 benzène
- l'hexabromobenzène
- le bromo-2 mésitylène
- le bromo-5 triméthyl-1,2,4 benzène
- le bromo-1-tert-butyl-4 benzène
- les bromostyrènes
- le bromo-2 chlorobenzène
- le bromo-3 chlorobenzène
- le bromo-4 chlorobenzène
- le bromo-2 chloro-5 toluène
- les bromodichlorobenzènes
- le bromo-5 fluoro-2 toluène
- les bromo (trifluorométhyl)benzènes
- les bromofluorobenzènes
- les bromodifluorobenzènes
- les bromotrifluorobenzènes
- les bromotétrafluorobenzènes
- le bromo-1 nitro-2 benzène
- le bromo-1 nitro-4 benzène
- le bromo-2 nitro-4 toluène
- le bromo-2 nitro-5 toluène
- le bromo-2 nitro-6 toluène
- le bromo-1 naphtalène
- le bromo-2 naphtalène
- le bromo-9 anthracène
- le bromo-1 méthyl-2 naphtalène
- le bromo-1 méthyl-4 naphtalène
- le bromo-2 méthoxy-6 naphtalène
- le bromo-2 anisole
- le bromo-3 anisole
- le bromo-4 anisole
- le dibromo-2,6 méthyl-4 anisole
- l'α,α,α trifluorobromoanisole
- les bromofluoroanisoles
- le bromo-4 phénétole
- le bromo-1 diméthoxy-2,4 benzène

- le bromo-1 diméthoxy-2,5 benzène
- l'acide bromo-2 benzoïque
- l'acide bromo-3 benzoïque
- l'acide bromo-4 benzoïque
- l'acide bromo-3 méthyl-4 benzoïque
- l'ester méthylique de l'acide (dibromo-3,5 acétamido-4) benzoïque
- l'ester éthylique de l'acide (dibromo-3,5 acétamido-4) benzoïque
- l'ester méthylique de l'acide (dibromo-3,5 méthoxy-4) benzoïque
- l'ester éthylique de l'acide (dibromo-3,5 méthoxy-4) benzoïque
- l'ester méthylique de l'acide (dibromo-3,5 éthoxy-4) benzoïque
- l'ester éthylique de l'acide (dibromo-3,5 éthoxy-4) benzoïque
- la bromo-2' acétophénone
- la bromo-3' acétophénone
- la bromo-4' acétophénone
- la bromo-2 acétanilide
- la bromo-3 acétanilide
- la bromo-4 acétanilide
- les bromométhylacétanilides
- les bromofluoroacétanilides
- la bromo-4 (trifluorométhyl)-2 acétanilide
- la bromo-N,N-diméthylacétanilide
- la bromo-4 chloro-2 acétanilide
- les bromodifluoroacétanilides
- le bromo-2 benzonitrile
- le bromo-3 benzonitrile
- le bromo-4 benzonitrile
- le bromo-2 benzamide
- le bromo-4 benzamide
- les bromothiophénols
- le bromo-4 diphényléther
- le bromo-4 benzophénone
- les biphénols mono- ou polybromés
- le bromo-4 nitro-3 biphényl
- le bromo-4 méthyl-3 pyrazole
- le bromo-4 diméthyl-3,5 pyrazole
- le bromo-3 furane
- le bromo-5 indole
- le bromo-2 thiophène
- le bromo-3 thiophène
- la bromo-2 pyridine
- la bromo-3 pyridine
- la bromo-4 pyridine
- le bromo-4 isoquinoléïne
- le bromo-4 (méthylènedioxy)-1,2 benzène

Parmi les composés répondant à la formule générale (II), l'invention s'applique tout particulièrement aux composés aromatiques mono- ou polyhalogénés suivants :

- le bromobenzène
- les dibromobenzènes
- les tribromobenzènes
- le tétrabromo-1,2,4,5 benzène
- l'hexabromobenzène
- les bromostyrènes
- le bromo-2 chlorobenzène
- les bromofluorobenzènes
- les bromodifluorobenzènes
- les bromotrifluorobenzènes
- les bromotétrafluorobenzènes
- le bromo-2 anisole

– le bromo-3 anisole
– le bromo-4 anisole
– le dibromo-2,6 méthyl-4 anisole
– l'acide bromo-2 benzoïque
– l'acide bromo-3 benzoïque
– l'acide bromo-4 benzoïque
– l'ester méthylique de l'acide (dibromo-3,5 acétamido-4) benzoïque
– l'ester éthylique de l'acide (dibromo-3,5 acétamido-4) benzoïque
– l'ester méthylique de l'acide (dibromo-3,5 méthoxy-4) benzoïque
– l'ester éthylique de l'acide (dibromo-3,5 méthoxy-4) benzoïque
– l'ester méthylique de l'acide (dibromo-3,5 éthoxy-4) benzoïque
– l'ester éthylique de l'acide (dibromo-3,5 éthoxy-4) benzoïque
– la bromo-4 (trifluorométhyl)-2 acétanilide
– les biphényls mono- ou polybromés
– le bromo-2 thiophène
– le bromo-3 thiophène
– la bromo-2 pyridine
– la bromo-3 pyridine
– la bromo-4 pyridine

Un mode de réalisation préféré de l'invention consiste en un procédé de préparation de composés aromatiques alkoxylés porteurs d'au moins un groupe hydroxyle de formule générale (Ia) :

$$(HO)_m - Ro - (O-R)_n \qquad (Ia)$$

dans laquelle :

– m et n sont des nombres entiers supérieurs ou égaux à 1 avec, de préférence, m + n compris entre 2 et 6,
– $R_o$ et R ayant la signification donnée précédemment,

procédé qui consiste à faire réagir un composé aromatique porteur d'au moins un atome d'halogène et d'au moins un groupe hydroxyle répondant à la formule générale (IIa) :

$$(HO)_m - Ro - (X)_n \qquad (IIa)$$

dans laquelle :

– m et n sont des nombres entiers supérieurs ou égaux à 1 avec, de préférence, m + n compris entre 2 et 6,
– X représente un atome d'iode, de brome ou de chlore,
– $R_o$ ayant la signification donnée précédemment,

- et un alcoolate de métal alcalin ou alcalino-terreux de formule générale (III) :

$$M^{w+} [ O - R ]_w^- \qquad (III)$$

dans laquelle :

– M représente un métal alcalin ou alcalino-terreux,
– w représente la valence du métal alcalin ou alcalino-terreux,
– R ayant la signification donnée précédemment,

en présence d'un catalyseur au cuivre qui est le cuivre et/ou un composé du cuivre,
procédé caractérisé par le fait que l'on opère en présence d'une quantité efficace d'un co-catalyseur choisi parmi les isocyanures organiques, les isocyanates et les esters d'alcyne-dioïques.

A titre d'exemples de composés aromatiques porteurs d'au moins un atome d'halogène et d'au moins un groupe hydroxyle répondant à la formule générale (IIa), on peut citer plus particulièrement :

– le bromo-2 phénol
– le bromo-3 phénol
– le bromo-4 phénol
– le bromo-1 naphtol-2
– le bromo-6 naphtol-2
– le bromo-2 méthyl-4 phénol
– le bromo-4 diméthyl-2,6 phénol

– le bromo-4 diméthyl-3,5 phénol
– le dibromo-2,6 méthyl-4 phénol
– le bromo-2-p-crésol
– le bromo-2 chloro-4 phénol
– le bromo-4 chloro-2 phénol
– le bromo-4 chloro-6-o-crésol
– les bromofluorophénols
– l'acide bromo-4 dihydroxy-3,5 benzoïque
– l'acide bromo-5 dihydroxy-2,4 benzoïque
– les bromo bis-phénols
– les bromo isopropylidène-4,4' bis-phénols

L'alcoolate de métal alcalin ou alcalino-terreux qui intervient dans le procédé de l'invention répond à la formule (III) dans laquelle w est égal à 1 ou 2 et R représente :

1° - un radical alkyle, alcényle, alcadiényle, alcynyle linéaire ou ramifié ayant, de préférence, moins de 6 atomes de carbone,

2° - un radical cycloalkyle ou cycloalcényle ayant, de préférence, de 5 à 7 atomes de carbone et l'on peut citer en particulier le radical cyclohexyle,

3° - un radical aliphatique acyclique, saturé ou insaturé, linéaire ou ramifié tel que précisé sous 1°, porteur d'un substituant cyclique. Par cycle, on entend un cycle carbocyclique saturé, insaturé ou aromatique. Comme exemple, on peut mentionner le radical benzyle.

Parmi les alcoolates précités, on met en oeuvre de préférence dans le procédé de l'invention, les alcoolates de métaux alcalins et plus particulièrement les alcoolates de sodium ou de potassium des alcanols primaires ou secondaires ayant 1 à 4 atomes de carbone conviennent particulièrement bien.

Les plus fréquemment utilisés sont le méthylate de sodium et l'éthylate de sodium.

Un mode de mise en oeuvre préférentiel de l'invention réside dans un procédé de préparation d'un composé aromatique alkoxylé entrant dans la définition de la formule (I) et répondant plus particulièrement à la formule générale (Ib) :

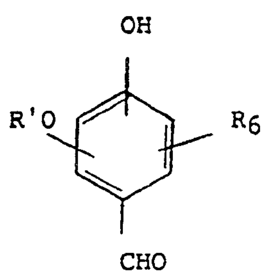

(Ib)

dans laquelle :

– R', $R_6$ identiques ou différents représentent un radical alkyle, linéaire ou ramifié, ayant de 1 à 4 atomes de carbone,

– $R_6$ pouvant représenter également un atome d'hydrogène, un radical alkoxy, linéaire ou ramifié ayant de 1 à 4 atomes de carbone, un radical hydroxyle.

Le procédé de l'invention de préparation d'un composé aromatique alkoxylé répondant à la formule générale (Ib) est un procédé qui consiste à faire réagir :

- un composé aromatique porteur d'au moins un atome d'halogène et d'au moins un groupe hydroxyle entrant dans la définition générale de la formule (II) qui est un halogéno hydroxy benzaldéhyde répondant à la formule générale (IIb) :

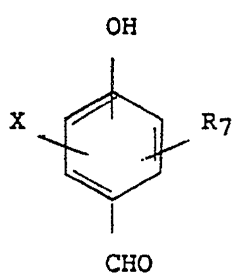

(IIb)

dans laquelle :

– X représente un atome d'iode, de brome ou de chlore,

– $R_7$ représente un atome d'hydrogène, un atome d'iode, de brome, un atome de chlore, un radical alkyle ayant de 1 à 4 atomes de carbone, un radical alkoxy ayant de 1 à 4 atomes de carbone, un radical hydroxyle,

dans ladite formule (IIb), le radical hydroxyle pouvant être en position ortho—, méta— ou para— de la fonction aldéhyde,

- avec un alcoolate de métal alcalin ou alcalino-terreux ayant de 1 à 4 atomes de carbone,

en présence d'un catalyseur au cuivre qui est le cuivre et/ou un composé du cuivre,

procédé caractérisé par le fait que l'on opère en présence d'une quantité efficace d'un co-catalyseur choisi parmi les isocyanures organiques, les isocyanates et les esters d'alcyne-dioïques.

Comme exemples de substrats mis en oeuvre préférentiellement, on peut citer plus particulièrement les composés répondant à la formule générale (IIb) dans laquelle :

- Groupe I :
  - le radical OH est en position para par rapport à la fonction CHO,
  - l'atome d'halogène X est en position ortho par rapport au radical OH,
  - le radical $R_7$ est en position ortho par rapport au radical OH.
- Groupe II :
  - le radical OH est en position ortho par rapport à la fonction CHO,
  - l'atome d'halogène X est en position para par rapport au radical OH,
  - le radical $R_7$ est en position ortho par rapport au radical OH.
- Groupe III :
  - le radical OH est en position ortho par rapport à la fonction CHO,
  - l'atome d'halogène X est en position ortho par rapport au radical OH,
  - le radical $R_7$ est en position para par rapport au radical OH,
- Groupe IV :
  - le radical OH est en position méta par rapport à la fonction CHO,
  - l'atome d'halogène X est en position ortho par rapport au radical OH,
  - le radical $R_7$ est en position para par rapport au radical OH.
- Groupe V :
  - le radical OH est en position méta par rapport à la fonction CHO,
  - l'atome d'halogène X est en position para par rapport au radical OH,
  - le radical $R_7$ est en position ortho par rapport au radical OH.

Les composés de formule (IIb) mis en oeuvre préférentiellement, répondent plus précisément aux formules suivantes :

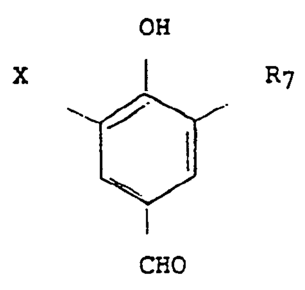

($IIb_1$)

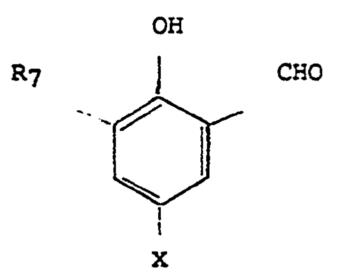

($IIb_2$)

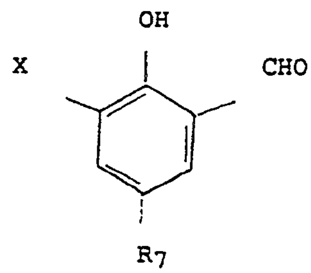

(IIb3)

dans lesquelles :

– X et $R_7$ ont les significations données précédemment.

Dans le procédé de l'invention, on met tout préférentiellement en oeuvre, un halogéno hydroxy benzaldéhyde répondant à la formule générale ($IIb_1$) dans laquelle X est un atome de brome et $R_7$ symbolise un radical méthoxy ou éthoxy.

A titre d'exemples d'halogéno hydroxy benzaldéhydes répondant à la formule (IIb) qui servent de substrats de départ dans le présent procédé, on peut citer plus précisément :

– le bromo-3 hydroxy-4 benzaldéhyde,
– le iodo-3 hydroxy-4 benzaldéhyde
– le dibromo-3,5 hydroxy-4 benzaldéhyde
– le diiodo-3,5 hydroxy-4 benzaldéhyde
– le bromo-5 méthoxy-3 hydroxy-4 benzaldéhyde
– le iodo-5 méthoxy-3 hydroxy-4 benzaldéhyde
– le bromo-5 éthoxy-3 hydroxy-4 benzaldéhyde
– le iodo-5 éthoxy-3 hydroxy-4 benzaldéhyde
– le bromo-3 dihydroxy-4,5 benzaldéhyde
– le iodo-3 dihydroxy-4,5 benzaldéhyde
– le bromo-3 dihydroxy-2,5 benzaldéhyde
– le iodo-3 dihydroxy-2,5 benzaldéhyde
– le bromo-2 hydroxy-4 benzaldéhyde
– le iodo-2 hydroxy-4 benzaldéhyde
– le bromo-4 hydroxy-3 benzaldéhyde
– le iodo-4 hydroxy-3 benzaldéhyde
– le bromo-3 hydroxy-2 benzaldéhyde
– le iodo-3 hydroxy-2 benzaldéhyde
– le bromo-5 hydroxy-2 benzaldéhyde
– le iodo-5 hydroxy-2 benzaldéhyde

Dans un mode préférentiel de mise en oeuvre du procédé de l'invention pour la préparation de composés aromatiques alkoxylés de formule (Ib), l'alcoolate de métal alcalin ou alcalino-terreux de formule (III) est de préférence un alcoolate de sodium ou potassium d'un alcanol primaire ou secondaire ayant de 1 à 4 atomes de carbone et, de préférence, le méthylate ou l'éthylate de sodium.

Conformément au procédé de l'invention, on fait réagir le composé aromatique porteur d'au moins un atome d'halogène et éventuellement d'un groupe hydroxyle répondant à la formule (II) avec un alcoolate de métal alcalin ou alcalino-terreux de formule (III), en présence d'un catalyseur au cuivre et d'un co-catalyseur choisi parmi les isocyanures organiques, les isocyanates et les esters d'alcyne-dioïques.

Les composés du cuivre servant de catalyseurs sont connus. Ce sont d'une manière générale tous les composés organiques ou inorganiques du cuivre I ou du cuivre II.

Le cuivre métal peut être utilisé, mais son action est plus lente car il faut préalablement qu'il se transforme en partie en cuivre I ou cuivre II.

A titre non limitatif, on peut citer comme composés du cuivre, le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule $ClCuOCH_3$, $Cu_2(OCH_3)_2(acac)_2$.

Les isocyanures organiques utilisés dans l'invention sont plus particulièrement les isocyanures de formule générale (IV) :

$$CN - R_8 \qquad (IV)$$

dans laquelle $R_8$ représente :

- un radical alkyle linéaire ou ramifié ayant 1 à 12 atomes de carbone,
- un radical cycloalkyle ayant 5 à 12 atomes de carbone,
- un radical cycloalkyle ayant 5 ou 12 atomes de carbone substitué par un ou deux radicaux alkyle ayant 1 à 12 atomes de carbone,
- un radical aryle ayant de 6 à 12 atomes de carbone,
- un radical aryle substitué par 1 ou 2 radicaux alkyle ayant 1 à 12 atomes de carbone,

Parmi ces isocyanures organiques, on utilise généralement les composés de formule (IVa) qui correspondent aux composés de formule (IV) dans laquelle $R_8$ représente :

- un radical alkyle linéaire ou ramifié ayant 2 à 8 atomes de carbone tels que éthyle, propyle, isopropyle, n-butyle, sec-butyle, isobutyle, tert-butyle, n-pentyle, n-hexyle, éthyl-2 hexyle, n-octyle,
- un radical cyclohexyle,
- un radical phényle,
- un radical benzyle ou phénéthyle.

A titre d'exemples non limitatifs de tels isocyanures organiques, on peut citer l'isocyanure d'éthyle, l'isocyanure de n-butyle, l'isocyanure de tertiobutyle, l'isocyanure de phényle et l'isocyanure de cyclohexyle.

Les isocyanates utilisés dans l'invention peuvent être des monoisocyanates ou des polyisocyanates aliphatiques, aromatiques ou cycloaliphatiques ainsi que leurs carbamates.

A titre d'exemples non limitatifs de tels isocyanates, on peut citer notamment le butylisocyanate, le diisocyanato-1,6 hexane, le diisocyanato-1,5 méthyl-2 pentane, le diisocyanato-1,4 éthyl-2 butane, l'isophorone-diisocyanate, l'hexyl-isocyanate, le phényl-isocyanate, le cyclohexyl-isocyanate, les toluène-diisocyanates.

Les esters d'acides alcyne-dioïques utilisés dans l'invention sont plus particulièrement les composés de formule générale (V) :

$$R_9-OOC-C \equiv C-COO-R_{10} \qquad (V)$$

dans laquelle $R_9$ et $R_{10}$, identiques ou différents, représentent :

- un radical alkyle, linéaire ou ramifié, ayant 1 à 12 atomes de carbone,
- un radical cycloalkyle ayant 5 à 12 atomes de carbone,
- un radical cycloalkyle ayant 5 à 12 atomes de carbone substitué par un ou deux radicaux alkyle ayant 1 à 12 atomes de carbone,
- un radical aryle ayant 6 à 12 atomes de carbone,
- un radical aryle substitué par 1 ou 2 radicaux alkyle ayant 1 à 12 atomes de carbone.

Parmi les esters d'acides alcyne-dioïques, on utilise de préférence les composés de formule (Va) qui correspondent aux composés de formule (Va) dans laquelle $R_9$ et $R_{10}$ représentent :

- un radical alkyle, linéaire ou ramifié, ayant 1 à 12 atomes de carbone,
- un radical cycloalkyle ayant 5 ou 6 atomes de carbone,
- un radical phényle,
- un radical phényle dont la partie alkyle comporte de 1 à 4 atomes de carbone.

A titre d'exemples non limitatifs de tels esters de formule (V), on peut citer notamment le butyne-2 dioate-1,4 de diméthyle, le butyne-2 dioate-1,4 de diéthyle, le butyne-2 dioate-1,4 de di-n-propyle, le butyne-2 dioate-1,4 de di-n-butyle, le butyne-2 dioate-1,4 de bis (éthyl-2 hexyle), le butyne-2 dioate-1,4 de diphényle, le butyne-2 dioate-1,4 de dicyclohexyle.

Conformément au procédé de l'invention, on fait réagir le composé aromatique porteur d'au moins un atome d'halogène et éventuellement d'un groupe hydroxyle répondant à la formule (II) avec un alcoolate de métal alcalin ou alcalino-terreux de formule (III), en présence d'un catalyseur au cuivre et d'un co-catalyseur choisi parmi les isocyanures organiques, les isocyanates et les esters d'alcyne-dioïques.

Le procédé de l'invention convient également lorsque le composé de formule (II) présente plus d'un atome d'halogène. Dans ce cas, on fera une polyalkoxylation, la seule condition étant uniquement d'adapter la quantité d'alcoolate de métal alcalin ou alcalino-terreux à mettre en oeuvre.

Selon le procédé de l'invention, l'alkoxylation du composé aromatique porteur d'au moins un atome d'halogène et éventuellement d'un groupe hydroxyle répondant à la formule (II) est conduite en milieu organique constitué, le plus souvent, par l'alcanol correspondant à l'alcoolate de métal alcalin ou alcalino-terreux utilisé.

On choisit, préférentiellement comme solvant, le méthanol ou l'éthanol.

Lorsque l'alcoolate de métal alcalin ou alcalino-terreux présente une condensation en carbone supérieure à 4 atomes de carbone, il est souhaitable de faire appel à un solvant inerte vis-à-vis de la réaction et de choisir, de préférence, un solvant aprotique polaire.

Comme exemples de solvants aprotiques polaires convenant à la mise en oeuvre du procédé de l'invention,

on peut mentionner, plus particulièrement les éthers et, plus précisément, les éthers diméthyliques dérivant de l'oxyde d'éthylène ou de l'oxyde de propylène tels que le diméthyléther de l'éthylèneglycol (ou diméthoxy-1,2 éthane), le diméthyl- éther du diéthylèneglycol (ou diméthoxy-1,5 oxa-3 pentane), le diméthoxy-1,8 dioxa-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,7 diméthyl-1,4 dioxa-3,6 octane.

On peut également utiliser plusieurs solvants.

Parmi tous les solvants précités, le diméthyléther de l'éthylèneglycol et le diméthyléther du diéthylèneglycol sont préférés.

La concentration du composé de formule (II) exprimée en poids dudit composé (II) par rapport au poids total composé (II) + solvant est généralement de 3 à 40% et, de préférence, de 10 à 30%.

La quantité d'alcoolate de métal alcalin ou alcalino-terreux utilisée est égale ou supérieure à la quantité stoechiométrique nécessaire, d'une part pour transformer le ou les atomes d'halogène en groupements alkoxy et d'autre part, si le noyau aromatique est porteur d'un ou plusieurs groupes OH, pour effectuer la salification du ou des groupes OH. Dans le cas où le composé de formule (II) est un composé phénolique, on le transforme en phénate de métal alcalin ou alcalino-terreux.

Généralement, l'alcoolate de métal alcalin ou alcalino-terreux est mis en oeuvre en une quantité correspondant à la quantité stoechiométrique nécessaire pour salifier les groupes hydroxyle plus une quantité égale de 1 fois à 5 fois, et de préférence de 1 à 3 fois, la quantité stoechiométrique nécessaire pour transformer le ou les atomes d'halogène en groupements alkoxy.

La concentration de l'alcoolate de métal alcalin ou alcalino-terreux est avantageusement supérieure à 1 mole/litre et, de préférence comprise entre 1 et 5 moles/litre.

Il est à noter que la borne supérieure ne présente aucun caractère critique.

De manière pratique, l'alcoolate de métal alcalin ou alcalino-terreux est formé in situ, en faisant réagir un excès d'alcanol avec le métal alcalin ou alcalino-terreux choisi.

La quantité de catalyseur au cuivre utilisée dans le procédé de l'invention peut varier très largement.

Habituellement, le rapport molaire catalyseur au cuivre/composé de formule (II) est de 1 à 50% et, de préférence, de 2% à 20%.

La quantité de composé insaturé, défini précédemment comme co-catalyseur, utilisée habituellement dans le procédé de l'invention, est telle que l'on ait un rapport molaire composé insaturé/catalyseur au cuivre de 1,0 à 20,0 et de préférence de 1,0 à 10,0, et encore plus préférentiellement de 1,0 à 5,0.

Cette quantité de composé insaturé peut également être exprimée par rapport au composé de formule (II), en ce qui concerne sa limite supérieure.

Ainsi, généralement on utilisera au plus 2 moles de composé insaturé pour 1 mole de composé de formule (II) et de préférence 1 mole de composé insaturé pour 1 mole de composé de formule (II).

La température de la réaction d'alkoxylation est généralement comprise entre 60°C et 220°C, et, de préférence entre 100°C et 180°C.

La pression n'est pas un paramètre critique en soi, mais pour atteindre les températures indiquées précédemment et ne pas perdre de solvant, le procédé est habituellement réalisé sous pression autogène.

Généralement, cette pression autogène du mélange réactionnel est inférieure ou égale à 5 MPa (50 bars).

La durée de la réaction d'alkoxylation peut varier largement entre 1 et 10 heures, de préférence entre 2 et 5 heures.

Comme mentionné précédemment, un autre objet de la présente invention est un procédé de préparation de composés aromatiques polyalkoxylés à partir des composés aromatiques alkoxylés obtenus selon le procédé d'alkoxylation de l'invention.

En effet, il a également été trouvé que dès lors que le produit obtenu en fin de réaction d'alkoxylation présentait également un groupe hydroxyle, celui-ci pouvait être pouvait soumis une à réaction de O-alkylation, par addition directement dans le milieu réactionnel, de l'agent d'alkylation.

Une variante d'exécution de l'invention réside, donc, en un procédé de préparation de composés aromatiques polyalkoxylés de formule générale (I) :

$$(R_1-O)_m - Ro - (O-R)_n \qquad (I)$$

dans laquelle :

– m et n sont des nombres entiers supérieurs ou égaux à 1 avec, de préférence, m + n compris entre 2 et 6,

– $R_0$ et R ayant la signification donnée précédemment,

– $R_1$ est un radical alkyle, linéaire ou ramifié ayant de 1 à 6 atomes de carbone,

procédé caractérisé par le fait que l'on effectue successivement les étapes suivantes :

1°- On fait réagir un composé aromatique porteur d'au moins un atome d'halogène et d'au moins un groupe hydroxyle répondant à la formule générale (IIa) :

$$(HO)_m - Ro - (X)_n \qquad (IIa)$$

dans laquelle :

– m et n sont des nombres entiers supérieurs ou égaux à 1 avec, de préférence, m+ n compris entre 2 et 6,

– X représente un atome d'iode, de brome ou de chlore,

– $R_o$ ayant la signification donnée précédemment,

- et un alcoolate de métal alcalin ou alcalino-terreux de formule générale (III) :

$$M^{w+} [ O - R ]_w^- \qquad (III)$$

dans laquelle :

– M représente un métal alcalin ou alcalino-terreux,

– w représente la valence du métal alcalin ou alcalino-terreux,

– R ayant la signification donnée précédemment,

en présence d'un catalyseur au cuivre qui est le cuivre et/ou un composé du cuivre et d'une quantité efficace d'un co-catalyseur choisi parmi les isocyanures organiques, les isocyanates et les esters d'alcyne-dioïques,

2° - On réalise la O-alkylation du ou des groupes hydroxyle du produit obtenu précédemment après alkoxylation, par addition directement dans le milieu réactionnel organique d'un agent d'alkylation choisi parmi les halogénures d'alkyle inférieurs, les dialkylsulfates, les dialkylcarbonates.

Un mode de mise en oeuvre préférentiel de l'invention réside dans un procédé de préparation d'un composé aromatique polyalkoxylé entrant dans la définition de la formule (I) et répondant plus particulièrement à la formule générale (Ib') :

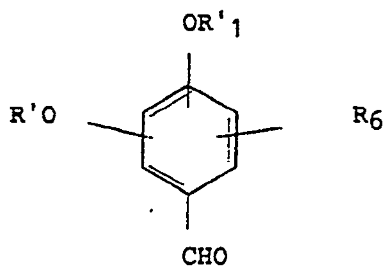

(Ib')

dans laquelle :

– R', $R'_1$, $R_6$ identiques ou différents représentent un radical alkyle, linéaire ou ramifié, ayant de 1 à 4 atomes de carbone,

– $R_6$ pouvant représenter également un atome d'hydrogène, un radical alkoxy, linéaire ou ramifié ayant de 1 à 4 atomes de carbone.

Le procédé selon l'invention, de préparation d'un composé aromatique polyalkoxylé répondant à la formule générale (Ib') comporte les étapes suivantes :

1° - On fait réagir un composé aromatique porteur d'au moins un atome d'halogène et d'au moins un groupe hydroxyle entrant dans la définition générale de la formule (II) qui est un halogéno hydroxy benzaldéhyde répondant à la formule générale (IIb) telle que définie auparavant, avec un alcoolate de métal alcalin ou alcalino-terreux ayant de 1 à 4 atomes de carbone, en présence d'un catalyseur au cuivre qui est le cuivre et/ou un composé du cuivre et d'une quantité efficace d'un co-catalyseur choisi parmi les isocyanures organiques, les isocyanates et les esters d'alcyne-dioïques,

2° - On réalise la O-alkylation du ou des groupes hydroxyle du produit obtenu précédemment après alkoxylation, par addition directement dans le milieu réactionnel organique d'un agent d'alkylation apportant le reste alkyle $R'_1$ ayant de 1 à 4 atomes de carbone.

Selon une variante du procédé de l'invention, on soumet le composé aromatique porteur d'au moins un atome d'halogène et d'au moins un groupe hydroxyle répondant à la formule (IIa), et, de préférence, à la formule (IIb), à une réaction d'alkoxylation en faisant réagir ledit composé avec l'alcoolate de métal alcalin ou alcalino-terreux, en présence d'un catalyseur au cuivre et d'un co-catalyseur de type insaturé, puis on soumet le composé aromatique ainsi obtenu porteur d'au moins un groupe alkoxy et d'au moins un groupe hydroxyle, à

une réaction de O-alkylation en le faisant réagir avec un agent d'alkylation.

Dans le cadre de la présente invention, on prépare, par exemple, un dialkoxy-3,4 et un trialkoxy-3,4,5 benzaldéhyde à partir respectivement d'un halogéno-3 hydroxy-4 benzaldéhyde et d'un halogéno-5 hydroxy-4 alkoxy-3 benzaldéhyde de formule (IIb) en soumettant les substrats de départ, d'abord à une réaction d'alkoxylation puis à une réaction de O-alkylation.

Dans le cas où le composé de formule (IIa) présente un ou plus d'un atome d'halogène et un ou plus d'un groupe hydroxyle, on fera respectivement une mono- ou poly-alkoxylation dans la première étape ou une mono- ou poly-O-alkylation dans la deuxième étape, la seule condition étant uniquement d'adapter les quantités de réactifs en fonction du nombre de groupes à substituer.

Dans une étape additionnelle du procédé de l'invention, on peut conduire la réaction de O-alkylation du composé aromatique précédemment obtenu qui est porteur d'au moins un groupe alkoxy et d'au moins un groupe hydroxyle.

A cet effet, on le met en contact avec un agent d'alkylation. On peut faire appel aux alkylsulfates de formule $R_1$-O-$SO_2$-O-$R_1$ ou aux alkylcarbonates de formule $R_1$-O-CO-O-$R_1$, dans lesdites formules, $R_1$ représente un radical alkyle, linéaire ou ramifié, ayant de 1 à 6 atomes de carbone.

Parmi les agents d'alkylation précités, le diméthylsulfate ou le diméthylcarbonate sont préférés.

Toutefois, on préfère selon l'invention choisir, à titre d'agent d'alkylation, un halogénure d'alkyle inférieur répondant à la formule générale (VI) :

$$R_1-X \qquad (VI)$$

dans ladite formule, X représente un atome de brome, de chlore ou d'iode et $R_1$ représente un radical alkyle linéaire ou ramifié ayant de 1 à 6 atomes de carbone.

Parmi les halogénures de formule (VI), on préfère mettre en oeuvre ceux répondant à la formule (VIa) dans laquelle X est un atome de chlore et $R'_1$ un radical alkyle linéaire ou ramifié ayant de 1 à 4 atomes de carbone.

Le plus souvent, on utilise un alogénure de méthyle ou un halogénure d'éthyle.

Parmi les halogénures, les chlorures, bromures et iodures sont généralement mis en oeuvre et plus spécifiquement encore le chlorure de méthyle, le chloroéthane, le bromure de méthyle et le bromoéthane.

En raison de leur plus faible coût, on préfère utiliser le chlorure de méthyle et le chloroéthane.

Une variante d'exécution de l'invention consiste à ajouter dans le milieu réactionnel, de préférence avant l'addition de l'agent d'alkylation, un sel sous forme d'iodure, afin d'avoir une cinétique plus élevée. Comme sels, on peut citer les iodures de métal alcalin, notamment de sodium, de potassium ou de lithium.

Généralement, la réaction de O-alkylation est enchaînée directement après la réaction d'alkoxylation, en introduisant l'agent d'alkylation à la température requise. Toutefois, dans certains cas, il peut être plus avantageux de maintenir le pH, à un pH compris entre 6 et 12 pendant la durée de la réaction.

Lorsque l'on opère à un pH inférieur à 6, on observe une diminution de la réaction de O-alkylation ainsi qu'un ralentissement de cette réaction.

D'une manière préférentielle, le pH du milieu est maintenu entre 9 et 11.

La régulation du pH peut être si nécessaire assurée par addition continue d'une base, de préférence, une solution aqueuse d'un hydroxyde de métal alcalin. Tous les hydroxydes de métal alcalin peuvent être utilisés. Cependant pour des considérations économiques, la soude est préférée.

Pour ce qui est de la quantité des réactifs à mettre en oeuvre dans cette étape du procédé de l'invention, on définit ci-après les quantités préférées.

La quantité d'agent d'alkylation engagée est fonction du nombre de groupes hydroxyle présents sur le noyau aromatique du produit obtenu précédemment, après alkoxylation. Elle est de préférence au moins égale à quantité stoechiométrique jusqu'à un excès pouvant atteindre 200%. Elle est, de préférence, égale à la quantité stoechiométrique.

A titre d'exemples, on précisera que le rapport molaire halogénure d'alkyle/alkoxy hydroxy benzaldéhyde varie de 1,0 à 3,0 et, de préférence, voisin de 1,0 et que le rapport molaire halogénure d'alkyle/alkoxy dihydroxy benzaldéhyde varie de 2,0 à 6,0 et, de préférence, voisin de 2,0.

La quantité de base est déterminée de telle sorte que le pH soit maintenu dans l'intervalle précité.

La quantité de sel sous forme d'iodure mis en oeuvre selon une autre variante préférée de l'invention peut également varier largement. Habituellement, le rapport molaire entre le sel sous forme d'iodure et l'agent d'alkylation varie entre 0,05 et 0,20 et se situe de préférence, aux environs de 0,10.

En ce qui concerne les conditions réactionnelles, la température de la réaction de O-alkylation n'est pas critique ; elle a une influence sur la cinétique de la réaction. Généralement, on réalise la réaction de O-alkylation entre 80°C et 200°C. De préférence, on opère à une température entre 100°C et 160°C.

La pression n'est pas critique. Elle a une incidence sur la cinétique de la réaction. Elle varie habituellement

entre la pression atmosphérique et 50 bars et se situe, de préférence, entre la pression atmosphérique et 20 bars.

La pression est habituellement créée par le solvant réactionnel et l'agent d'alkylation servant à la O-alkylation lorsqu'ils sont gazeux dans les conditions réactionnelles.

La durée de la réaction de O-alkylation est fonction notamment de la température réactionnelle. Elle varie le plus souvent entre 1 heure et 8 heures. Généralement, une durée de 1 à 4 heures est suffisante.

Selon un mode de réalisation pratique de l'invention, on peut mélanger tous les réactifs de la première étape à savoir le composé aromatique de formule (II), le catalyseur au cuivre et le co-catalyseur de type insaturé et après chauffage pendant la durée nécessaire à la réaction d'alkoxylation, ajouter dans le milieu réactionnel, l'agent d'alkylation éventuellement une base.

Un mode préféré de mise en pratique de l'invention consiste d'abord à préparer une solution de l'alcoolate de métal alcalin dans l'alcanol correspondant à raison de, par exemple, 20 à 50% en poids.

Dans l'appareillage, on charge sous atmosphère de gaz inerte, de préférence, l'azote, le composé aromatique de formule (II), le catalyseur au cuivre puis le co-catalyseur de type insaturé, puis l'on introduit la solution de l'alcoolate de métal alcalin.

On porte ensuite le milieu réactionnel à la température choisie entre 60°C et 220°C, de préférence, entre 100°C et 180°C, pendant la durée pré-définie.

On introduit ensuite l'agent d'alkylation, de préférence, l'halogénure d'alkyle qui peut être liquide ou gazeux et on injecte ou coule en parallèle la solution d'hydroxyde de métal alcalin, si celle-ci est nécessaire pour ajuster le pH dans la zone précitée.

On maintient le chauffage entre 80°C et 200°C, de préférence entre 100°C et 160°C, pendant la durée nécessaire à la réaction de O-alkylation.

En fin de réaction, on sépare le composé aromatique polyalkoxylé de formule (I) obtenu, selon les techniques classiques de séparation, soit par distillation, soit par extraction par un solvant approprié, par exemple, le toluène dans le cas du triméthoxy-3,4,5 benzaldéhyde.

Il est possible d'éliminer le catalyseur au cuivre du milieu réactionnel selon les traitements classiques, notamment par traitement acide.

Le procédé de l'invention permet de réaliser l'alkoxylation de tout substrat porteur d'un atome d'halogène et ceci, avec un très bon rendement.

Un autre avantage du procédé de l'invention est qu'il fait appel dans sa forme préférentielle, à un solvant courant, peu onéreux et facilement recyclable.

Le procédé de l'invention est parfaitement bien adapté à la préparation des alkoxybenzaldéhydes suivants :

- le triméthoxy-3,4,5 benzaldéhyde
- le diméthoxy-3,4 benzaldéhyde.
- le diéthoxy-3,4 benzaldéhyde
- l'éthoxy-3 méthoxy-4 benzaldéhyde

Le triméthoxy-3,4,5 benzaldéhyde qui peut être préparé selon le procédé de l'invention sert notamment pour la préparation de produits pharmaceutiques.

Un des avantages particulièrement intéressant du procédé de l'invention est qu'il permet d'effectuer la réaction d'alkoxylation sans qu'il soit nécessaire de protéger la fonction aldéhyde.

Un autre intérêt du procédé de l'invention est qu'il permet d'effectuer à la suite et dans le même réacteur, la réaction d'alkoxylation et de O-alkylation.

Dans le cas de la préparation du triméthoxy-3,4,5 benzaldéhyde, on effectue sa préparation à partir de bromo-5 hydroxy-4 méthoxy-3 benzaldéhyde, en milieu organique essentiellement homogène, sans séparation du produit intermédiaire alkoxylé, ce qui est très avantageux d'un point de vue industriel.

Les exemples qui suivent illustrent l'invention sans toutefois la limiter.

Dans les exemples, les abréviations suivantes signifient :

BHMB : bromo-5 hydroxy-4 méthoxy-3 benzaldéhyde

BHB : bromo-3 hydroxy-4 benzaldéhyde

syringaldéhyde : hydroxy-4 diméthoxy-3,5 benzaldéhyde

$$TT = \frac{\text{Nombre de moles de composé de formule (II) transformées}}{\text{Nombre de moles de composé de formule (II) introduites}} \%$$

$$RT = \frac{\text{Nombre de moles de composé de formule (I) formées}}{\text{Nombre de moles de composé de formule (II) transformées}} \%$$

$$RR = \frac{\text{Nombre de moles de composé de formule (I) formées}}{\text{Nombre de moles de composé de formule (II) introduites}} \%$$

## EXEMPLE 1

### Méthoxylation du bromo-5 hydroxy-4 méthoxy-3 benzaldéhyde

Dans un réacteur téfloné de 40 $cm^3$, muni d'un système de chauffage et d'une agitation, on charge :

- 2,22 g (0,0096 mol) de bromo-5-hydroxy-4-méthoxy-3 benzaldéhyde (BHMB)
- 2,16 g (0,040 mol) de méthylate de sodium
- 25 $cm^3$ de méthanol
- 0,099 g (0,001 mol) de chlorure cuivreux
- 0,4 $cm^3$ (0,0038 mol) d'isocyanure de n-butyle.

On chauffe sous agitation pendant 3 h à 125°C.

On refroidit à température ambiante, puis on dilue le mélange réactionnel par 80 $cm^3$ d'eau distillée. On ajuste ensuite à 4 le pH du mélange obtenu à l'aide d'acide sulfurique.

On filtre la partie insoluble et on dose par chromatographie liquide haute performance (CLHP) le BHMB restant et le syringaldéhyde (hydroxy-4 diméthoxy-3,5 benzaldéhyde) formé.

On obtient les résultats suivants :

- taux de transformation (TT) du BHMB : 100 %
- rendement (RT) en syringaldéhyde par rapport au BHMB transformé : 94 %

## EXEMPLE 2

### Méthoxylation du bromo-5 hydroxy-4 méthoxy-3 benzaldéhyde

On répète l'exemple 1, avec les mêmes charges, les mêmes conditions opératoires en remplaçant l'isocyanure de n-butyle par l'isocyanure de tertiobutyle.

On obtient les résultats suivants :

- taux de transformation (TT) du BHMB : 98 %
- rendement (RT) en syringaldéhyde : 99 %

## EXEMPLE 3

### Méthoxylation du bromo-5 hydroxy-4 méthoxy-3 benzaldéhyde

On répète l'exemple 1, avec les mêmes charges, les mêmes conditions opératoires, en remplaçant l'isocyanure de n-butyle par 0,476 g (0,004 mol) de phénylisocyanate et le chlorure cuivreux par 0,110 g (0,0005 mol) de carbonate de cuivre II basique de formule $CuCO_3,Cu(OH)_2$.

On obtient les résultats suivants :

- taux de transformation (TT) du BHMB : 95 %
- rendement (RT) en syringaldéhyde : 99 %

EXEMPLE 4

Méthoxylation du bromo-3 hydroxy-4 benzaldéhyde

Dans un réacteur téfloné de 40 $cm^3$, muni d'un système de chauffage et d'une agitation magnétique, on charge :
- 1,02 g (0,005 mol) de bromo-3 hydroxy-4 benzaldéhyde (BHB)
- 1,08 g (0,020 mol) de méthylate de sodium
- 10 $cm^3$ de méthanol
- 0,055 g (0,00025 mol) de carbonate de cuivre II basique de formule $CuCO_3$,$Cu(OH)_2$
- 0,2 $cm^3$ (0,0019 mol) d'isocyanure de n-butyle.

On chauffe 4 h 15 à 125°C sous agitation.

On refroidit à température ambiante, on dilue à l'eau distillée, on ajuste le pH du mélange réactionnel à 4 à l'aide d'acide sulfurique et on dose par CLHP.

On obtient les résultats suivants :

- taux de transformation (TT) du BHB : 75 %
- rendement (RT) en vanilline : 99 %

EXEMPLE 5

Méthoxylation du bromo-3 hydroxy-4 benzaldéhyde

On répète l'exemple 4, avec les mêmes charges, les mêmes conditions opératoires, en remplaçant l'isocyanure de n-butyle par l'isocyanure de tertiobutyle.

On obtient les résultats suivants :

- taux de transformation (TT) du HBB : 88 %
- rendement (RT) en vanilline : 93 %

EXEMPLE 6

Méthoxylation du bromo-5 hydroxy-4 méthoxy-3 benzaldéhyde

On répète l'exemple 1, avec les mêmes charges, les mêmes conditions opératoires, en remplaçant l'isocyanure de n-butyle par 0,577 g (0,004 mol) de butyne-2 dioate-1,4 de diméthyle.

On obtient les résultats suivants :

- taux de transformation (TT) du BHMB : 100 %
- rendement (RT) en syringaldéhyde : 97 %

EXEMPLE 7

Méthoxylation du bromobenzène

Dans un réacteur téfloné de 40 $cm^3$, muni d'un système de chauffage et d'une agitation, on charge :
- 1,57 g (0,010 mol) de bromobenzène
- 2,71 g (0,015 mol) d'une solution de méthylate de sodium à 30% dans le méthanol
- 0,110 g (0,0005 mol) de carbonate de cuivre II basique de formule $CuCO_3$,$Cu(OH)_2$
- 0,44 g (0,004 mol) d'isocyanure de cyclohexyle

On chauffe 5 heures 30 à 125°C.

Après traitement du milieu réactionnel comme dans l'exemple 1, on dose par chromatographie en phase gazeuse 0,83 g d'anisole, ce qui correspond à un rendement (RR) de 76,4%.

EXEMPLE 8

Méthoxylation du bromobenzène

On répète l'exemple 7, mais en remplaçant l'isocyanure de cyclohexyle par 0,57 g (0,004 mol) de butyne-2 dioate-1,4 de diméthyle.

On obtient 0,96 g d'anisole, ce qui correspond à un rendement (RR) de 88,8%.

EXEMPLE 9

Méthoxylation du bromo-5 hydroxy-4 méthoxy-3 benzaldéhyde suivie d'une méthylation

Dans un réacteur de 3,9 litres en acier inoxydable, muni d'un système de chauffage et d'une agitation, on charge sous atmosphère d'azote :

- 170,5 g (0,738 mol) de bromo-5 hydroxy-4 méthoxy-3 benzaldéhyde (BHMB)
- 162 g de méthylate de sodium
- 1785 $cm^3$ (1428 g) de méthanol
- 8,3 g (0,0377 mol) de carbonate de cuivre II basique de formule $CuCO_3,Cu(OH)_2$
- 29,7 g (0,300 mol) d'isocyanure de n-butyle,

On chauffe sous agitation, pendant 4 heures, à 125°C, sous pression autogène, afin de réaliser la réaction de méthoxylation.

On refroidit le mélange réactionnel à 120°C puis l'on charge 150 g de chlorure de méthyle.

On maintient le mélange réactionnel à 120°C pendant 3 heures.

On refroidit à température ambiante.

On sépare par filtration la partie insoluble.

On dose par chromatographie liquide haute performance, le BHMB n'ayant pas réagi et le triméthoxy-3,4,5 benzaldéhyde obtenu.

Les résultats obtenus sont les suivants :

- taux de transformation du BHMB ($TT_{BHMB}$ %) = 100 %
- rendement en TMBA ($RT_{TMBA}$ %) = 82 %
- rendement en syringaldéhyde (RT syringaldéhyde %) = 16 %

**Revendications**

1. - Procédé de préparation de composés aromatiques polyalkoxylés de formule générale (I) :

$$(R_1-O)_m-\ Ro\ -(O-R)_n \qquad (I)$$

dans laquelle :

- m et n sont des nombres entiers,
  - n est un nombre entier supérieur ou égal à 1,
  - m est un nombre entier supérieur ou égal à 0,
  - la somme m + n étant comprise, de préférence, entre 1 et 6,
- Ro représente un radical cyclique aromatique ayant au moins 5 atomes dans le cycle, éventuellement substitué, et représentant au moins l'un des radicaux suivants :
  - un radical carbocyclique aromatique, monocyclique ou polycyclique,
  - un radical hétérocyclique aromatique, monocyclique ou polycyclique comportant au moins un des hétéroatomes O, N et S,
- R représente un radical hydrocarboné ayant de 1 à 12 atomes de carbone, qui peut être un radical

aliphatique acyclique, saturé ou insaturé, linéaire ou ramifié ; un radical cycloaliphatique, saturé ou insaturé, monocyclique ou polycyclique ; un radical cycloaliphatique— ou arylaliphatique, saturé ou insaturé, linéaire ou ramifié,

– $R_1$ est un radical alkyle, linéaire ou ramifié ayant de 1 à 6 atomes de carbone,

procédé qui consiste à faire réagir :

- un composé aromatique porteur d'au moins un atome d'halogène et, éventuellement, d'un groupe hydroxyle répondant à la formule générale (II) :

$$(HO)_m - Ro - (X)_n \qquad (II)$$

dans laquelle :

– m et n sont des nombres entiers,
  - n est un nombre entier supérieur ou égal à 1,
  - m est un nombre entier supérieur ou égal à 0,
  - la somme m + n étant comprise, de préférence, entre 1 et 6,

– X représente un atome d'iode, de brome ou de chlore,

– $R_o$ représente un radical cyclique aromatique ayant au moins 5 atomes dans le cycle, éventuellement substitué, et représentant au moins l'un des radicaux suivants :
  - un radical carbocyclique aromatique, monocyclique ou polycyclique,
  - un radical hétérocyclique aromatique, monocyclique ou polycyclique comportant au moins un des hétéroatomes O, N et S, - et un alcoolate de métal alcalin ou alcalino-terreux de formule générale (III) :

$$M^{W+} [ O - R ]_w^- \qquad (III)$$

dans laquelle :

– M représente un métal alcalin ou alcalino-terreux,

– w représente la valence du métal alcalin ou alcalino-terreux,

– R représente un radical hydrocarboné ayant de 1 à 12 atomes de carbone, qui peut être un radical aliphatique acyclique, saturé ou insaturé, linéaire ou ramifié ; un radical cycloaliphatique, saturé ou insaturé, monocyclique ou polycyclique ; un radical cycloaliphatique— ou arylaliphatique, saturé ou insaturé, linéaire ou ramifié,

en présence d'un catalyseur au cuivre qui est le cuivre et/ou un composé du cuivre,

procédé caractérisé par le fait que l'on opère en présence d'une quantité efficace d'un co-catalyseur qui est un composé insaturé choisi parmi les isocyanures organiques, les isocyanates et les esters des alcyne-dioïques.

2. - Procédé selon la revendication 1 caractérisé par le fait que le composé aromatique de formule (II) est un composé aromatique porteur d'au moins un atome d'halogène et d'au moins un groupe hydroxyle répondant à la formule générale (IIa) :

$$(HO)_m - Ro - (X)_n \qquad (IIa)$$

dans laquelle :

– m et n sont des nombres entiers supérieurs ou égaux à 1 avec, de préférence, m + n compris entre 2 et 6,

– X est un atome d'iode, de brome ou de chlore,

– Ro ayant la signification donnée précédemment,

3. - Procédé selon l'une des revendications 1 et 2 caractérisé par le fait que le composé aromatique porteur d'au moins un atome d'halogène et éventuellement d'un groupe hydroxyle, répond à la formule générale (II) dans laquelle Ro symbolise :

1°) un radical carbocyclique aromatique, monocyclique ou polycyclique,

2°) un radical hétérocyclique aromatique, monocyclique ou polycyclique,

3°) un radical divalent constitué par un enchaînement de groupements, tels que définis aux paragraphes 1 et/ou 2 liés entre eux :

• par un lien valentiel
• par un radical alkylène ou alkylidène ayant de 1 à 4 atomes de carbone, de préférence un radical méthylène ou isopropylidène,
• par l'un des groupes suivants :

$-O-$ , $-CO-$ ,

$-S-$ , $-SO-$ , $-SO_2-$ ,

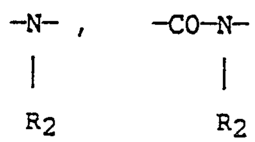

dans ces formules, $R_2$ représentant un atome d'hydrogène ou un radical alkyle ayant de 1 à 4 atomes de carbone, un radical cyclohexyle ou phényle.

4. - Procédé selon l'une des revendications 1 à 3 caractérisé par le fait que le composé aromatique porteur d'au moins un atome d'halogène et, éventuellement, d'un groupe hydroxyle répond à la formule générale (II) dans laquelle $R_o$ est substitué par un ou plusieurs substituants tels que :
• un radical de formule —$R_3$-OH dans laquelle le radical $R_3$ représente un lien valentiel ou un radical hydrocarboné divalent, linéaire ou ramifié, saturé ou insaturé ayant de 1 à 4 atomes de carbone, tel que par exemple, méthylène, éthylène, propylène, isopropylène, isopropylidène,
• un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
• un radical alcényle linéaire ou ramifié ayant de 2 à 6 atomes de carbone, de préférence, de 2 à 4 atomes de carbone, tel que vinyle, allyle,
• un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
• un groupe —CHO,
• un groupe acyle ayant de 2 à 6 atomes de carbone,
• un radical de formule —$R_3$-COOH, $R_3$ ayant la signification donnée précédemment,
• un radical de formule —$R_3$-$COOR_4$ dans laquelle $R_3$ a la signification donnée précédemment et $R_4$ représente un radical alkyle, linéaire ou ramifié, ayant de 1 à 6 atomes de carbone,
• un radical de formule —$R_3$-$NH_2$ avec un groupe $NH_2$ N-protégé, $R_3$ ayant la signification donnée précédemment,
• un radical de formule —$R_3$-$N(R_5)_2$ dans laquelle $R_3$ a la signification donnée précédemment et les radicaux $R_5$ identiques ou différents représentent un atome d'hydrogène ou un radical alkyle, linéaire ou ramifié, ayant de 1 à 6 atomes de carbone,
• un radical de formule —$R_3$-CO-$N(R_5)_2$, $R_3$ et $R_5$ ayant la signification donnée précédemment,
• un radical de formule —$R_3$-Z dans laquelle $R_3$ a la signification donnée précédemment et Z symbolise un atome d'halogène X, tel que précédemment défini, un atome de fluor ou un groupe $CF_3$.

5. - Procédé selon l'une des revendications 1 à 4 caractérisé par le fait que le composé aromatique porteur d'au moins un atome d'halogène et éventuellement d'un groupe hydroxyle répond à la formule générale (II) dans laquelle $R_o$ est un noyau benzénique.

6. - Procédé selon la revendication 1 caractérisé par le fait que le composé aromatique porteur d'au moins un atome d'halogène répondant à la formule générale (II) est choisi parmi :
– le bromobenzène
– les dibromobenzènes
– les tribromobenzènes
– le tétrabromo-1,2,4,5 benzène
– l'hexabromobenzène

- les bromostyrènes
- le bromo-2 chlorobenzène
- les bromofluorobenzènes
- les bromodifluorobenzènes
- les bromotrifluorobenzènes
- les bromotétrafluorobenzènes
- le bromo-2 anisole
- le bromo-3 anisole
- le bromo-4 anisole
- le dibromo-2,6 méthyl-4 anisole
- l'acide bromo-2 benzoïque
- l'acide bromo-3 benzoïque
- l'acide bromo-4 benzoïque
- l'ester méthylique de l'acide (dibromo-3,5 acétamido-4) benzoïque
- l'ester éthylique de l'acide (dibromo-3,5 acétamido-4) benzoïque
- l'ester méthylique de l'acide (dibromo-3,5 méthoxy-4) benzoïque
- l'ester éthylique de l'acide (dibromo-3,5 méthoxy-4) benzoïque
- l'ester méthylique de l'acide (dibromo-3,5 éthoxy-4) benzoïque
- l'ester éthylique de l'acide (dibromo-3,5 éthoxy-4) benzoïque
- la bromo-4 (trifluorométhyl)-2 aniline
- les biphényls mono- ou polybromés
- le bromo-2 thiophène
- le bromo-3 thiophène
- la bromo-2 pyridine
- la bromo-3 pyridine
- la bromo-4 pyridine

7. - Procédé selon la revendication 2 caractérisé par le fait que le composé aromatique porteur d'au moins un atome d'halogène et d'au moins un groupe hydroxyle répondant à la formule générale (IIa) est choisi parmi :
   - le bromo-2 phénol
   - le bromo-3 phénol
   - le bromo-4 phénol
   - le bromo-1 naphtol-2
   - le bromo-6 naphtol-2
   - le bromo-2 méthyl-4 phénol
   - le bromo-4 diméthyl-2,6 phénol
   - le bromo-4 diméthyl-3,5 phénol
   - le dibromo-2,6 méthyl-4 phénol
   - le bromo-2-p-crésol
   - le bromo-2 chloro-4 phénol
   - le bromo-4 chloro-2 phénol
   - le bromo-4 chloro-6-o-crésol
   - les bromofluorophénols
   - l'acide bromo-4 dihydroxy-3,5 benzoïque
   - l'acide bromo-5 dihydroxy-2,4 benzoïque
   - les bromo bis-phénols
   - les bromo isopropylidène-4,4' bis-phénols

8. - Procédé selon l'une des revendications 1 et 2 caractérisé par le fait que le composé aromatique porteur d'au moins un atome d'halogène et d'au moins un groupe hydroxyle est un halogéno hydroxy benzaldéhyde répondant à la formule générale (IIb) :

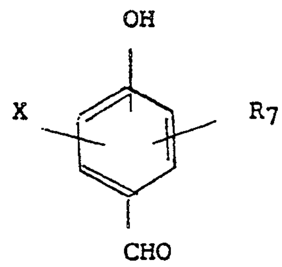

(IIb)

dans laquelle :

– X représente un atome d'iode, de brome ou de chlore,

– $R_7$ représente un atome d'hydrogène, un atome d'iode, de brome, un atome de chlore, un radical alkyle ayant de 1 à 4 atomes de carbone, un radical alkoxy ayant de 1 à 4 atomes de carbone, un radical hydroxyle,

dans ladite formule (IIb), le radical hydroxyle pouvant être en position ortho—, méta— ou para— de la fonction aldéhyde.

**9.** - Procédé selon la revendication 8 caractérisé par le fait que l'halogéno hydroxy benzaldéhyde répond à la formule générale (IIb) dans laquelle :

- Groupe I :
  – le radical OH est en position para par rapport à la fonction CHO,
  – l'atome d'halogène X est en position ortho par rapport au radical hydroxyle OH,
  – le radical $R_7$ est en position ortho par rapport au radical OH.
- Groupe II :
  – le radical OH est en position ortho par rapport à la fonction CHO,
  – l'atome d'halogène X est en position para par rapport au radical OH,
  – le radical $R_7$ est en position ortho par rapport au radical OH.
- Groupe III :
  – le radical OH est en position ortho par rapport à la fonction CHO,
  – l'atome d'halogène X est en position ortho par rapport au radical OH,
  – le radical $R_7$ est en position para par rapport au radical OH.
- Groupe IV :
  – le radical OH est en position méta par rapport à la fonction CHO,
  – l'atome d'halogène X est en position ortho par rapport au radical OH,
  – le radical $R_7$ est en position para par rapport au radical OH.
- Groupe V :

– le radial OH est en position méta par rapport à la fonction CHO,

– l'atome d'halogène X est en position para par rapport au radical OH,

– le radical $R_7$ est en position ortho par rapport au radical OH.

**10.** - Procédé selon l'une des revendications 9 et 10 caractérisé par le fait que l'halogéno hydroxy benzaldéhyde répond à l'une des formules suivantes :

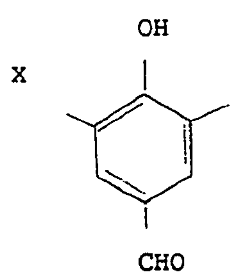

($IIb_1$)

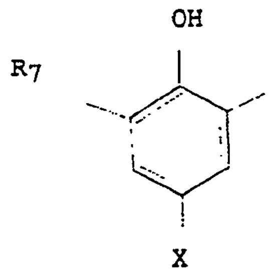

(IIb$_2$)

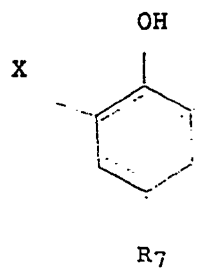

(IIb$_3$)

dans lesquelles :

– X et $R_7$ ont les significations données précédemment.

**11.** - Procédé selon l'une des revendications 9 à 11 caractérisé par le fait que l'halogéno hydroxy benzaldéhyde est choisi parmi :

– le bromo-3 hydroxy-4 benzaldéhyde,
– le iodo-3 hydroxy-4 benzaldéhyde
– le dibromo-3,5 hydroxy-4 benzaldéhyde
– le diiodo-3,5 hydroxy-4 benzaldéhyde
– le bromo-5 méthoxy-3 hydroxy-4 benzaldéhyde
– le iodo-5 méthoxy-3 hydroxy-4 benzaldéhyde
– le bromo-5 éthoxy-3 hydroxy-4 benzaldéhyde
– le iodo-5 éthoxy-3 hydroxy-4 benzaldéhyde
– le bromo-3 dihydroxy-4,5 benzaldéhyde
– le iodo-3 dihydroxy-4,5 benzaldéhyde
– le bromo-3 dihydroxy-2,5 benzaldéhyde
– le iodo-3 dihydroxy-2,5 benzaldéhyde
– le bromo-2 hydroxy-4 benzaldéhyde
– le iodo-2 hydroxy-4 benzaldéhyde
– le bromo-4 hydroxy-3 benzaldéhyde
– le iodo-4 hydroxy-3 benzaldéhyde
– le bromo-3 hydroxy-2 benzaldéhyde
– le iodo-3 hydroxy-2 benzaldéhyde
– le bromo-5 hydroxy-2 benzaldéhyde
– le iodo-5 hydroxy-2 benzaldéhyde

**12.** - Procédé selon l'une des revendications 1 à 11 caractérisé par le fait que l'alcoolate de métal alcalin ou alcalino-terreux répond à la formule générale (III) dans laquelle R représente un radical alkyle, alcényle, alcadiényle, alcynyle linéaire ou ramifié ayant de préférence moins de 6 atomes de carbone ou un radical cyclohexyle ou benzyle.

**13.** - Procédé selon l'une des revendications 1 à 12 caractérisé par le fait que l'alcoolate de métal alcalin est un alcoolate de sodium ou de potassium d'un alcanol primaire ou secondaire ayant 1 à 4 atomes de carbone.

**14.** - Procédé selon l'une des revendications 1 à 13 caractérisé par le fait que l'alcoolate de métal alcalin est le méthylate de sodium ou l'éthylate de sodium.

**15.** - Procédé selon l'une des revendications 1 à 14 caractérisé par le fait que le catalyseur est un composé organique ou inorganique du cuivre I ou du cuivre II, choisi parmi le chlorure cuivreux, le chlorure cuivrique, le carbonate de cuivre II basique, le nitrate cuivreux, le nitrate cuivrique, le sulfate cuivrique, l'acétate cuivrique, le trifluorométhylsulfonate cuivrique, l'hydroxyde cuivrique, le picolinate de cuivre II, le méthylate de cuivre I, le méthylate de cuivre II, le chélate de cuivre II de la 8-quinoléine, les composés de cuivre de formule $ClCuOCH_3$, $Cu_2(OCH_3)_2(acac)_2$.

**16.** - Procédé selon l'une des revendications 1 à 15 caractérisé par le fait que le co-catalyseur est un isocyanure de formule générale (IV) :

$$CN - R_8 \qquad (IV)$$

dans laquelle $R_8$ représente :
- un radical alkyle linéaire ou ramifié ayant 1 à 12 atomes de carbone,
- un radical cycloalkyle ayant 5 à 12 atomes de carbone,
- un radical cycloalkyle ayant 5 à 12 atomes de carbone substitué par un ou deux radicaux alkyle ayant 1 à 12 atomes de carbone,
- un radical aryle ayant 6 à 12 atomes de carbone,
- un radical aryle substitué par 1 ou 2 radicaux alkyle ayant 1 à 12 atomes de carbone.

**17.** - Procédé selon la revendication 16 caractérisé par le fait que le co-catalyseur est un isocyanure de formule générale (IVa) :

$$CN - R_8 \qquad (IVa)$$

dans laquelle $R_8$ représente :
- un radical alkyle linéaire ou ramifié ayant 2 à 8 atomes de carbone,
- un radical cyclohexyle,
- un radical phényle,
- un radical benzyle ou phénéthyle.

**18.** - Procédé selon l'une des revendications 16 et 17 caractérisé par le fait que le co-catalyseur est un isocyanure choisi parmi l'isocyanure d'éthyle, l'isocyanure de n-butyle, l'isocyanure de tertiobutyle, l'isocyanure de phényle et l'isocyanure de cyclohexyle.

**19.** - Procédé selon l'une des revendications 1 à 15 caractérisé par le fait que le co-catalyseur est un monoisocyanate ou un polyisocyanate aliphatique, aromatique ou cycloaliphatique ou leur carbamate.

**20.** - Procédé selon la revendication 19 caractérisé par le fait que le co-catalyseur est un isocyanate choisi parmi le butylisocyanate, le diisocyanato-1,6 hexane, le diisocyanato-1,5 méthyl-2 pentane, le diisocyanato-1,4 éthyl-2 butane, l'isophorone-diisocyanate, l'hexyl-isocyanate, le phényl-isocyanate, le cyclohexylisocyanate, les toluène-diisocyanates.

**21.** - Procédé selon l'une des revendications 1 à 15 caractérisé par le fait que le co-catalyseur est un ester d'un acide alcyne-dioïque de formule générale (V) :

$$R_9-OOC-C \equiv C-COO-R_{10} \qquad (V)$$

dans laquelle $R_9$ et $R_{10}$, identiques ou différents, représentent :
- un radical alkyle, linéaire ou ramifié, ayant 1 à 12 atomes de carbone,
- un radical cycloalkyle ayant 5 à 12 atomes de carbone,
- un radical cycloalkyle ayant 5 à 12 atomes de carbone substitué par un ou deux radicaux alkyle ayant 1 à 12 atomes de carbone,
- un radical aryle ayant de 6 à 12 atomes de carbone,
- un radical aryle substitué par 1 ou 2 radicaux alkyle ayant 1 à 12 atomes de carbone.

22. - Procédé selon la revendication 21 caractérisé par le fait que le co-catalyseur est un ester d'un acide alcyne-dioïque de formule générale (Va) :

$$R_9\text{-OOC-C} \equiv \text{C-COO-}R_{10} \qquad \text{(Va)}$$

dans laquelle $R_9$ et $R_{10}$, identiques ou différents, représentent :
- un radical alkyle, linéaire ou ramifié, ayant 1 à 12 atomes de carbone,
- un radical cycloalkyle ayant 5 ou 6 atomes de carbone,
- un radical phényle,
- un radical phényle dont la partie alkyle comporte de 1 à 4 atomes de carbone.

23. - Procédé selon l'une des revendications 21 et 22 caractérisé par le fait que le co-catalyseur est un ester d'acides alcyne-dioïques choisi parmi le butyne-2 dioate-1,4 de diméthyle, le butyne-2 dioate-1,4 de diéthyle, le butyne-2 dioate-1,4 de di-n-propyle, le butyne-2 dioate-1,4 de di-n-butyle, le butyne-2 dioate-1,4 de bis (éthyl-2 hexyle), le butyne-2 dioate-1,4 de diphényle, le butyne-2 dioate-1,4 de dicyclohexyle.

24. - Procédé selon l'une des revendications 1 à 23 caractérisé par le fait que l'alcoolate de métal alcalin ou alcalino-terreux est mis en oeuvre en une quantité correspondant à la quantité stoechiométrique nécessaire pour salifier les groupes hydroxyle plus une quantité égale de 1 fois à 5 fois, et de préférence de 1 à 3 fois, la quantité stoechiométrique nécessaire pour transformer le ou les atomes d'halogène en groupements alkoxy.

25. - Procédé selon la revendication 1 à 24 caractérisé par le fait que la concentration de l'alcoolate de métal alcalin ou alcalino-terreux est supérieure à 1 mole/litre et, de préférence comprise entre 1 et 5 moles/litre.

26. - Procédé selon l'une des revendications 1 à 25 caractérisé par le fait que le rapport molaire composé du cuivre/composé de formule (II) est de 1% à 50% et, de préférence, de 2% à 20%.

27. - Procédé selon l'une des revendications 1 à 26 caractérisé par le fait que la réaction est conduite dans un solvant constitué par l'alcanol correspondant à l'alcoolate de métal alcalin ou alcalino-terreux utilisé.

28. - Procédé selon la revendication 27 caractérisé par le fait que la concentration initiale de composé de formule (II) par rapport au mélange composé de formule (II)/solvant est de 3% à 40% en poids par poids et, de préférence, de 10% à 30%.

29. - Procédé selon l'une des revendications 1 à 28 caractérisé par le fait que le rapport molaire co-catalyseur/composé au cuivre est de 1,0 à 20,0 et de préférence de 1,0 à 10,0, et encore plus préférentiellement de 1,0 à 5,0.

30. - Procédé selon l'une des revendications 1 à 29 caractérisé par le fait que l'on opère la réaction d'alkoxylation à une température de 60°C à 220°C et, de préférence, de 100°C à 180°C.

31. - Procédé selon l'une des revendications 1 à 5,7 à 30 caractérisé par le fait que l'on réalise, dans une étape suivante, la O-alkylation du ou des groupes hydroxyle du produit obtenu précédemment par réaction d'un composé aromatique porteur d'au moins un atome d'halogène et d'au moins un groupe hydroxyle de formule (IIa) avec un alcoolate de métal alcalin ou alcalino-terreux de formule (III), en présence d'un catalyseur et d'un co-catalyseur tels que définis, à une réaction de O-alkylation, par addition directement dans le milieu réactionnel organique d'un agent d'alkylation choisi parmi les halogénures d'alkyle inférieurs, les dialkylsulfates, les dialkylcarbonates.

32. - Procédé selon la revendication 31 caractérisé par le fait que l'agent alkylation dans la réaction de O-alkylation est le diméthylsulfate ou le diméthylcarbonate.

33. - Procédé selon la revendication 31 caractérisé par le fait que l'agent d'alkylation est un halogénure d'alkyle inférieur répondant à la formule générale (VI) :

$$R_1\text{-X} \qquad \text{(VI)}$$

dans ladite formule, X représente un atome de brome, de chlore ou d'iode et $R_1$ représente un radical alkyle linéaire ou ramifié ayant de 1 à 6 atomes de carbone.

**34.** - Procédé selon la revendication 33 caractérisé par le fait que l'agent d'alkylation est le chlorure de méthyle, le chloroéthane, le bromure de méthyle et le bromoéthane.

**35.** - Procédé selon l'une des revendications 31 à 34 caractérisé par le fait que la quantité d'halogénure d'alkyle exprimée par rapport à la quantité de composé de formule (II) varie de la quantité stoechiométrique à un excès pouvant atteindre 200%, de préférence égale à la quantité stoechiométrique.

**36.** - Procédé selon l'une des revendications 31 à 35 caractérisé par le fait que l'on introduit avant l'agent d'alkylation, un sel sous forme d'iodure de métal alcalin, de préférence de sodium.

**37.** - Procédé selon l'une des revendications 31 à 36 caractérisé par le fait que l'on réalise la réaction de O-alkylation à un pH de mélange réactionnel compris entre 6 et 12, de préférence, entre 9 et 11.

**38.** - Procédé selon l'une des revendications 31 à 37 caractérisé par le fait que l'on opère la réaction de O-alkylation entre 80°C et 200°C et, de préférence, entre 100°C et 160°C.

**39.** - Procédé selon l'une des revendications 1 à 38 caractérisé par le fait que le composé aromatique mono-ou polyalkoxylé de formule (I) est l'un des composés suivants :

- le méthoxybenzène
- le diméthoxy-1,2 benzène
- le méthoxy-2 phénol
- le méthoxy-4 phénol
- le triméthoxy-3,4,5 benzaldéhyde
- le diméthoxy-3,4 benzaldéhyde
- le diéthoxy-3,4 benzaldéhyde
- l'éthoxy-3 méthoxy-4 benzaldéhyde